# EUROPEAN PATENT SPECIFICATION

(11) **EP 1 522 259 B1**
(45) Date of publication and mention of the grant of the patent: **20.02.2008**
(21) Application number: 04022672.2
(22) Date of filing: 23.09.2004
(51) Int. Cl.: A61B 5/053, G01G 19/50

(54) **Body type determining apparatus and method for determining a body type**
Vorrichtung und Verfahren zur Bestimmung des Körpertyps
Dispositif et procédé pour la détermination du type du corps

(30) Priority: 08.10.2003 JP 2003349162
(43) Date of publication of application: 13.04.2005
(73) Proprietor: TANITA CORPORATION, Tokyo (JP)
(72) Inventor: Sato, Hitoshi, Itabashi-ku Tokyo (JP)
(74) Representative: Grünecker, Kinkeldey, Stockmair & Schwanhäusser Anwaltssozietät

(56) References cited:
- EP-A- 1 201 187
- DATABASE MEDLINE [Online] US NATIONAL LIBRARY OF MEDICINE (NLM), BETHESDA, MD, US; July 2003 (2003-07), KYLE URSULA G ET AL: "Body composition interpretation. Contributions of the fat-free mass index and the body fat mass index." XP008041494 Database accession no. NLM12831945 & NUTRITION (BURBANK, LOS ANGELES COUNTY, CALIF.) 2003 JUL-AUG, vol. 19, no. 7-8, July 2003 (2003-07), pages 597-604, ISSN: 0899-9007

## Description

The present Invention relates to a body type determining apparatus and a method for determining a body type, and In particular an apparatus which measures the body fat mass and lean mass of a body based on a bioelectrical impedance method. More specifically, it relates to determination of a body type by the apparatus.

Among conventional body type determining apparatuses, JP 10-192258 discloses an apparatus that classifies body types such as an athlete type having a high body weight and a low body fat percentage and an unobvious obese type having a low body weight and a high body fat percentage visually on a matrix, generally by displaying the relationship between a body mass index (hereinafter referred to as "BMI") and a body fat percentage on the matrix.

However, the above body type determining apparatuses do not take into account a lean mass. A significant portion of the lean mass is made up of a muscle amount. Since it has an influence on a basal metabolic rate which influences accumulation of body fat, it is an important element in determination of a body type. For example, the balance between a lean mass and a body fat mass at the time of dieting is not expressed in the above matrix-based display of BMI and a body fat percentage.

For this reason, EP 1201187 A1 discloses a body type determining apparatus adopting a matrix-based display mode useful for determination of a body type and determination of the effect of dieting by calculating a determination standard of LMI or FMI for each BMI based on the generally used proper range and obese range of a body fat percentage by use of FMI (Fat Mass Index: Body Fat Mass/ (Body Height)²) which is an index of body fat and LMI (Lean Mass Index: Lean Mass/ (Body Height)²) which is an index of lean mass.

However, even the above matrix-based display using LMI and FMI with respect to BMI may have differences from values measured from actual subjects because it is based on the theoretical proper and obese ranges of a body fat percentage. That is, BMI and a body fat percentage show a certain level of correlation, and in actual subjects, body fat percentages are also low when BMI values are low, and body fat percentages are also high when BMI values are high. However, when the above determination standards with the fixed ranges of a body fat percentage are used in determining, for example, an obese subject showing a high BMI value, LMI tends to be determined to be lower than an actual value, and FMI tends to be determined to be higher than an actual value. On the other hand, in determination of a subject showing a low BMI value, LMI may be determined to be a high value and FMI may be determined to be a low value.

Further, in the above cases, the proportion of body fat mass or lean mass is roughly determined, i.e., merely visually displayed by matrix-based display, and the proportion of body fat mass or lean mass is not clearly indicated in numerical values.

It is an objective of the present invention to provide a body type determining apparatus and a method for determining a body type allowing a more specific and adaptable comparison for the subject whose body type is to be determined.

According to an apparatus aspect of the present invention, said objective is solved by the combination of features of independent claim 1.

According to a method aspect, the aforementioned objective is solved by a method for determining a body type according to the wording of Independent claim 5.

Preferred embodiments of the present invention are laid down in the subclaims.

In the following, the present invention is explained in greater detail by means of embodiments thereof in conjunction with the accompanying drawings, wherein:
Fig. 1 is an external view of a body type determining apparatus of Example 1.
Fig. 2 is an electrical block diagram of Example 1.
Fig. 3 is a flowchart illustrating the operations of Example 1.
Fig. 4 is a graph illustrating the relationship between BMIand FMI using normal adult males as a population.
Fig. 5 is a graph illustrating the relationship between BMI and LMI using normal adult males as a population.
Fig. 6 is an external view of a body type determining apparatus of Example 2.
Fig. 7 is an example of display of the present example.

The following examples comprise an input unit, an impedance measuring unit, a calculation unit, a standard setting unit, and a body type determining unit, wherein the Input unit inputs personal physical data, the impedance measuring unit measures a bioelectrical Impedance, the calculation unit calculates a body mass Index and a body composition Index based on the personal physical data and the bioelectrical impedance, the standard setting unit sets a normal value obtained from a regression formula based on measured data of the body mass Index and the body composition Index, as a body type determination standard, and the body type determining unit determines a body type based on the body type determination standard.

The body composition index is at least one of BMI and LMI. FMIand BMI are used to determine the proportions of the body fat mass and lean mass of a body, respectively. Further, the standard setting unit sets a number of body type determination standards for different objects for comparison and allows a subject to select a body type determination standard suited for the subject. Based on the body type determination standard, the proportions of body fat mass and lean mass are determined in numerical values with good accuracy.

### [Example 1]

In Example 1, the proportions of body fat mass and lean mass are determined based on normal values obtained from regression formulae of FMI and LMI with respect to BMI, and determinations of a variety of body types are made possible by setting the regression formulae for each object for comparison.

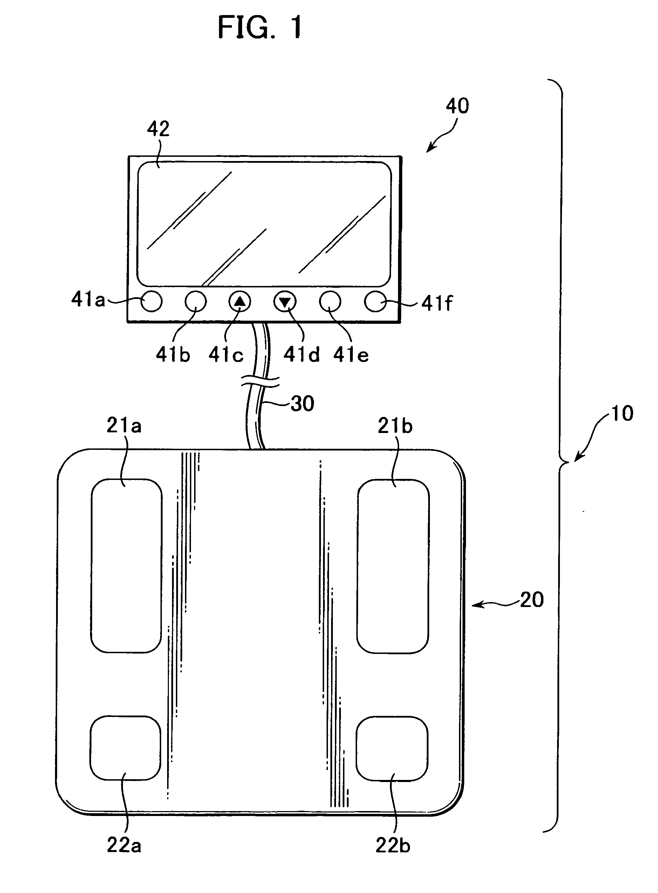

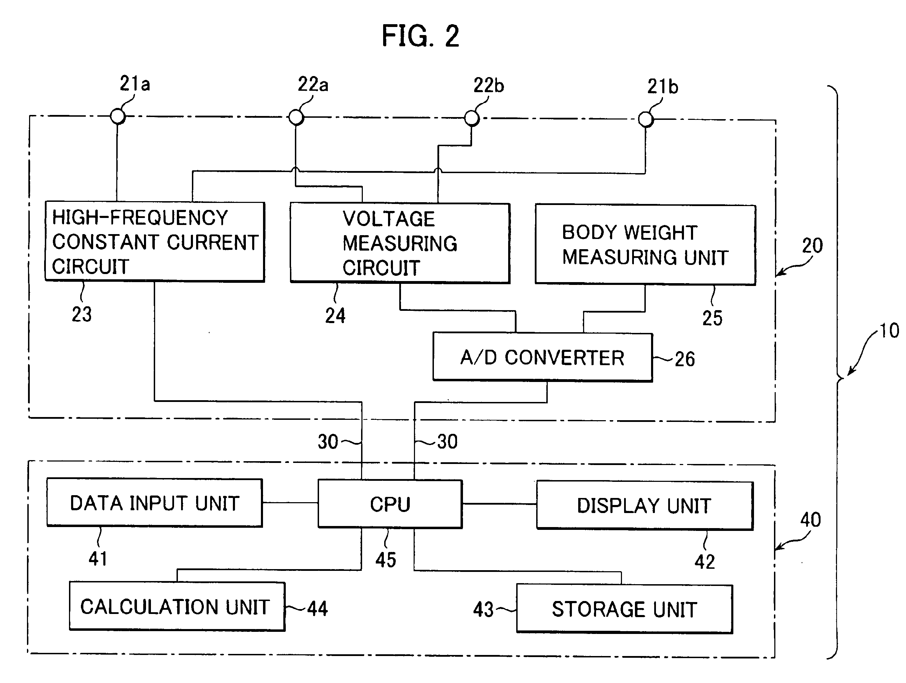

First, the constitution of the present Example 1 will be described by use of Figs. 1 and 2. Fig. 1 is an external front view of a body type determining apparatus, and Fig. 2 is an electrical block diagram of the body type determining apparatus.

As shown in Fig. 1, a body type determining apparatus 10 comprises a scale-incorporated bioelectrical impedance meter 20 and a control box 40 which are connected to each other via electric cables 30. Further, on the top surface of the bioelectrical impedance meter 20, constant current applying electrodes 21a and 21b and voltage measuring electrodes 22a and 22b are provided. Further, on the front face of the control box 40, operation keys comprising a power switch 41a, a measurement key 41b, an UP key 41c, a DOWN key 41d, a setting key 41e and a determination standard selecting key 41f and a display section 42 are provided.

Further, as shown in Fig. 2, in the bioelectrical impedance meter 20, the constant current applying electrodes 21a and 21b are connected to a HIGH-FREQUENCY constant current generating circuit 23, and the voltage measuring electrodes 22a and 22b are connected to a voltage measuring circuit 24. Further, it incorporates a body weight measuring unit 25, and the body weight measuring unit 25 as well as the voltage measuring circuit 24 are connected to an A/D converter 26 which converts an analog signal into a digital signal.

Further, the HIGH-FREQUENCY constant current circuit 23 and the A/D converter 26 are connected to a CPU 45 which controls calculations, determinations, display and storage of various data in the control box 40 via the electric cables 30. The CPU 45 is connected to a data input unit 41 which inputs data by means of the operation keys, a display unit 42 which displays the results of calculations and determinations in numerical values or as graphs, a storage unit 43 which stores a number of regression formulae preset as determination standards and various data, and a calculation unit 44 which sets body type determination standards by calculations of various data and calculations of normal values by the regression formulae and determines body types.

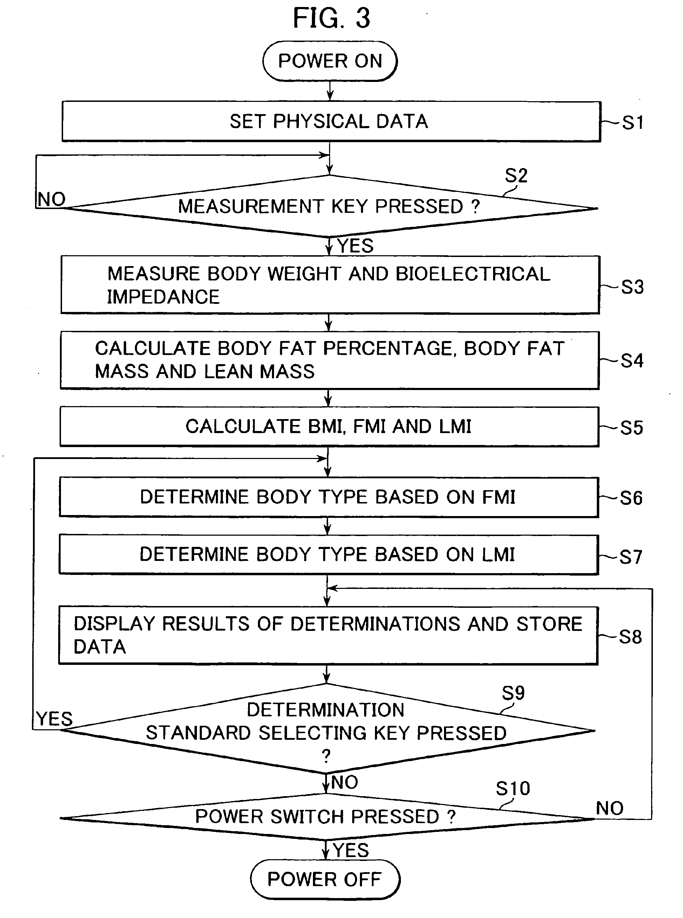

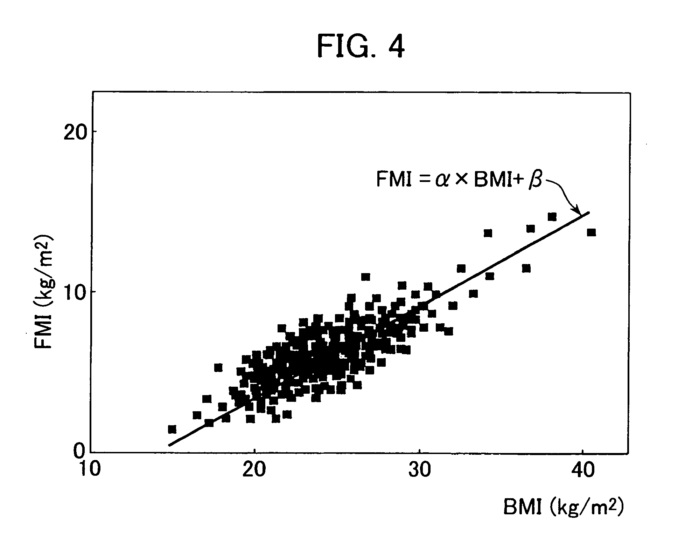

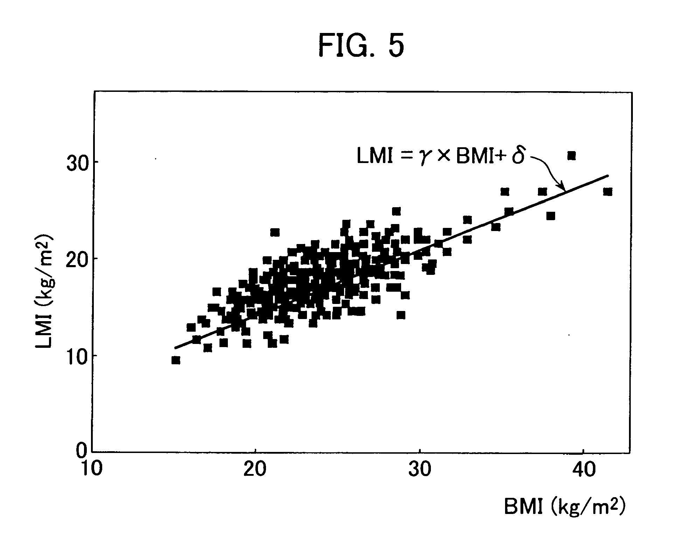

Next, the operation of the present Example 1 will be described by use of Figs. 3 to 5. Fig. 3 is a flowchart illustrating the operations of the body type determining apparatus 10, and Figs. 4 and 5 are graphs illustrating regression formulae which are statistically determined from measured data obtained by using normal adult males as a population. Fig. 4 is a graph illustrating the relationship between FMI and BMI which represents a determination standard for a body fat mass, and Fig. 5 is a graph illustrating the relationship between LMI and BMI which represents a determination standard for a lean mass.

In Fig. 3, when the power of the body type determining apparatus 10 is turned on at the press of the power switch 41a of the control box 40, the CPU 45 displays an instruction urging a subject to set physical data on the display unit 42 in STEP S1. The physical data are a body height, gender and age. The subject changes values displayed on the display unit 42 by use of the UP key 41c or the DOWN key 41d and sets each of the data in turn by use of the setting key 41e.

After completion of the setting of the physical data, the CPU 45 displays an instruction urging the subject to start measurements of a body weight and a bioelectrical impedance by pressing the measurement key 41b on the display unit 42 and determines whether the measurement key 41b has been pressed in STEP S2. When it has not been pressed, the CPU 45 repeats STEP S2, while when it has been pressed, the CPU 45 proceeds to STEP S3 to measure a body weight and a bioelectrical impedance value in accordance with known measurement methods. In subsequent STEP S4, a body fat percentage, a body fat mass and a lean mass are calculated from the above measured body weight and bioelectrical impedance value, and in STEP S5, the above BMI, FMI and LMI are calculated from the above body height, body weight, body fat mass and lean mass.

In STEP S6 and STEP S7, determination of a body type is made in the following manner. First, in STEP S6, a regression formula which is a determination standard for body fat which is preset in the storage unit 43 and obtained from FMI and BMI is retrieved. A number of such regression formulae are set according to, e.g., genders and ages, and an appropriate regression formula is automatically selected and retrieved based on the gender and age set in setting of physical data in STEP S1. For example, when the subject is a male of 30's, a regression formula prepared by using normal adult males as a population is read in. The regression formula is represented as, e.g., FMI = α × BMI + β wherein α and β are constants, as shown in Fig. 4. In the above calculation unit 44, the normal FMI value of the normal adult males with respect to BMI is calculated from this regression formula, and how much larger or smaller the body fat mass is than the standard can be determined in numerical values by showing the percentage of displacement of the above calculated FMI from the normal value defined as a standard value. That is, the proportion (%) of the body fat mass based on the standard can be represented by (FMI - normal FMI value) x 100/(normal FMI value).

Similarly, in STEP S7, the proportion of the lean mass based on a normal LMI value defined as a standard can be determined by showing the percentage of displacement of the above calculated LMI from the normal value, based on a regression formula prepared by using normal adult males as a population and represented by LMI = γ x BMI + δ (γ and δ are constants) as shown in Fig. 5. That is, the proportion (%) of the lean mass based on the standard is represented by (LMI - normal LMI value) x 100/(normal LMI value).

In STEP S8, the above determined proportions (%) of the body fat mass and the lean mass based on the standards are displayed in numerical values on the display unit 42 as the results of the determinations, and the data obtained by the above measurements and calculations are stored in the storage unit 43.

In STEP S9, it is determined whether the determination standard selecting key 41f has been pressed. While the regression formulae prepared by using normal adult males as a population are automatically selected as determination standards according to gender and age in the above STEPS S6 and S7, a number of determination standards set for more specific objects for comparison can be selected manually in STEP S9. The objects for comparison are classified by, for example, races, the types of athletes or ages which are more specific than notions such as elderly people and children, and regression formulae corresponding to these objects for comparison are stored in the storage unit 43.

More specifically, a list of the above more specific objects for comparison is displayed, together with the determination results displayed on the display unit 42. The subject selects an object for comparison from the list of the objects for comparison by use of the above determination standard selecting key 41f. Thereby, for example, when the subject is an athlete, determination of the body type of the subject as an athlete can be made by selecting an appropriate object for comparison from objects for comparison which are classified according to the types of sports. Further, by setting a determination standard prepared by using top athletes as a population for each type of sports, a self-training effect with top athletes as a target can be determined.

Further, when the subject is an elderly person, the body type of the subject can be expressed as muscle age or the like by use of determination based on a standard for the young as well as determination based on a standard for people of the same age as the subject.

Thus, when the determination standard selecting key 41f is pressed, the CPU 45 returns to STEP S6 so as to determine the body type based on a selected object for comparison. When the determination standard selecting key 41f is not pressed, the CPU 45 proceeds to STEP S10 so as to determine whether the power switch 41a has been pressed. If the power switch 41a is not pressed, the CPU 45 returns to STEP S8 and continues displaying the results, while if the power switch 41a is pressed, the CPU 45 turns off the power of the apparatus, thereby completing the whole operation.

In the present Example 1, the scale-incorporated bioelectrical impedance meter 20 and the control box 40 are connected to each other via the electric cables 30. However, data may be exchanged between them by wireless communication using infrared light, an electromagnetic wave or the like, or the bioelectrical impedance meter 20 and the control box 40 may be integrated.

### [Example 2]

Example 2 is a combination of the above body type determining apparatus 10 of Example 1 and a body height meter capable of measuring and automatically inputting a body height.

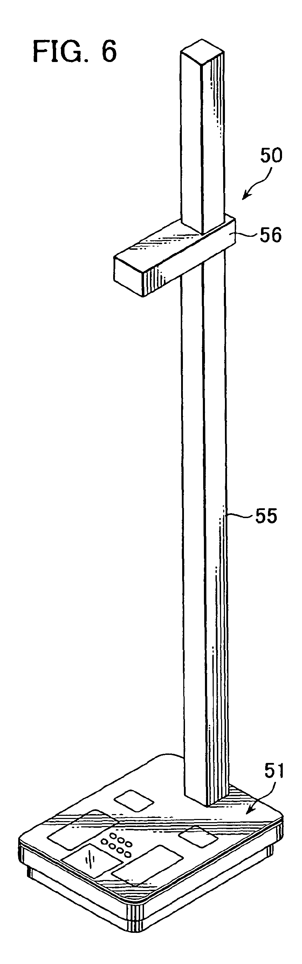

The constitution of Example 2 will be described by use of Fig. 6. A body-height-meter-incorporated body type determining apparatus 50 comprises a control-box-incorporated body type determining device 51 which has the control box 40 in the scale-incorporated bioelectrical impedance meter 20 shown in Fig. 1 in the first example, a pole 55, and a cursor 56 which measures a body height by moving up or down along the pole 55.

Further, its operation procedures are similar to those of Example 1 shown in the flowchart of Fig. 3. In Example 1, a body height is also manually input in numerical values in addition to gender and age in STEP S1, and in subsequent STEP S3, a body weight and a bioelectrical impedance are measured. Meanwhile, in Example 2, a body height is not input and only gender and age are input manually in STEP S1, and in STEP S3, the body height is also measured and input in addition to measurements of a body weight and a bioelectrical impedance. STEPS S4 to S10 are carried out in the same manner as in Example 1 using the measured body height.

Although the regression formula of FMI and BMI and the regression formula of LMI and BMI are presented as linear regression in STEPS S6 and S7 in the flowchart of Fig. 3 in the present example, they may be regression formulae represented by a logarithmic curve or an exponential curve.

Further, in the present example, the normal values obtained from the above regression formulae are used as determination standards, and the proportions (%) of body fat mass and lean mass based on the standards are displayed as determination results. However, it is also possible that, for example, in the relationship between FMI and BMI, variations in FMI data with respect to the normal value obtained from the above regression formula are set as a proper range or abnormal range with respect to the above standard value by use of a percentile value, standard deviation or Z score based on the standard deviation and used as a determination standard. The same applies to LMI in setting a determination standard.

Further, it is also possible that a proper range and an abnormal range are set by multiplying the above normal value which is a determination standard value by a preset given coefficient so as to determine a body type.

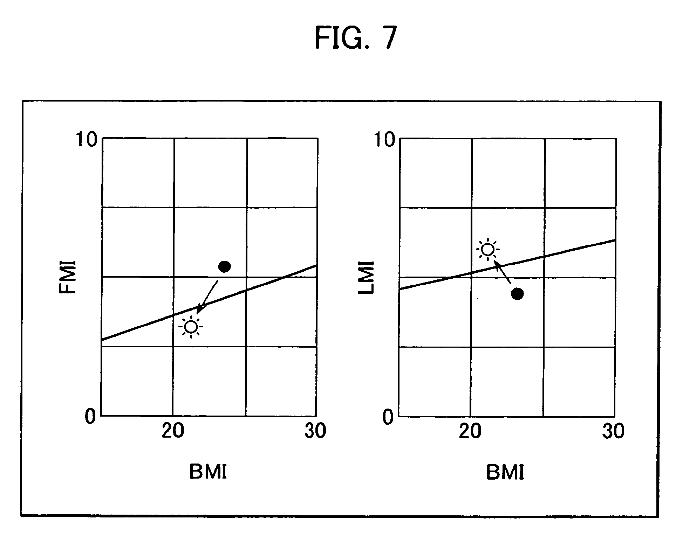

In STEP S8, although the results of the determinations are displayed in numerical values as the proportions (%) of body fat mass and lean mass with respect to the normal values, it is also possible to display the relationship between FMI and BMI and the relationship between LMI and BMI as graphs as exemplified in Fig. 7. In that case, the proportions from the normal values can be displayed visually by displaying regression lines represented by the regression formulae used as determination standards together with the proportions. Further, the degrees of changes with respect to the determination standards can be indicated as vectors by also plotting the results of past measurements as indicated by black dots in the drawing, whereby the effect of dieting or training can be shown in a more easily understandable manner.

Further, in graph display, when the display unit 42 is capable of dot matrix display, the graph can be made easier to see by calculating the middle point between a normal value and a measured value and automatically enlarging a certain range including the measured point or the calculated point as the center thereof.

## Claims

1. A body type determining apparatus comprising:
an input unit (41) for personal physical data of a subject whose body type is to be determined;
an impedance measuring unit for measuring a bioelectrical impedance of the subject;
a calculation unit (44) for calculating a body mass index (BMI) and a body composition index (FMI, LMI) based on the personal physical data and the bioelectrical impedance of the subject;
a storage unit (43) for pre-storing a plurality of regression formulae, said regression formulae corresponding to populations as objects for comparison, said populations being classified by physical data;
a standard setting unit for setting a normal value obtained from a regression formula based on measured body mass index data and body composition index data, which is selected from the plurality of regression formulae, as a body type determination standard;
a body type determining unit for determining the body type of the subject based on a difference between the normal value obtained from the selected regression formula and the calculated body composition index (FMI, LMI) of the subject; and
a determination standard selecting key (41f), which allows a manual selection of another population as a more specific object for comparison if operated, and wherein a regression formula corresponding to a population as object for comparison according to gender and age of the subject, whose body type is to be determined, is automatically selected if the determination standard selecting key (41f) is not operated where the apparatus is configured for determining the body type on the basis of the automatically selected regression formula, followed by expecting an input from the subject by means of the determination standard selecting key (41f) and upon said input from the subject determining the body type on the basis of a regression formula corresponding to the manually selected population.

2. A body type determining apparatus according to claim 1, wherein the calculation unit (44) calculates the body mass index (BMI) and at least one of a fat mass index (FMI) and a lean mass index (LMI) based on the personal physical data and the bioelectrical impedance of the subject.

3. A body type determining apparatus according to claim 1 or 2, wherein the standard setting unit sets a number of body type determination standards for different objects for comparison.

4. A body type determining apparatus according to one of claims 1 to 3, wherein the body type determining unit sets a proper range or an abnormal range of a body type based on at least one statistical technique out of a percentile value or a standard deviation of the body composition index based on the normal value or a Z-score based on the standard deviation, and determines the body type of the subject by a range to which the body composition index belongs.

5. A method for determining a body type comprising:
inputting, by an input unit (41), personal physical data of a subject whose body type is to be determined;
measuring a bioelectrical impedance of the subject by an impedance measuring unit;
calculating a body mass index (BMI) and a body composition index (FMI, LMI) based on the personal physical data and the bioelectrical impedance of the subject by a calculation unit (44);
pre-storing a plurality of regression formulae by a storage unit (43), said regression formulae corresponding to populations, said populations being as objects for comparison classified by physical data;
setting a normal value obtained from a regression formula based on measured body mass index data and body composition index data, which is automatically selected from the plurality of regression formulae according to gender and age of the subject if a determination standard selecting key (41f) is not operated, as a body type determination standard;
determining the body type of the subject based on a difference between the normal value obtained from the selected regression formula and the calculated body composition index (FMI, LMI) of the subject by a body type determining unit; and
selecting another population as a more specific object for comparison if the determination standard selecting key (41f) is operated where the body type is determined on the basis of the automatically selected regression formula, followed by a step of expecting an input from the subject by means of the determination standard selecting key (41f) and a step of determining the body type on the basis of a regression formula corresponding to the manually selected population upon said input from the subject.

## Patentansprüche

1. Körpertyp-Bestimmungsvorrichtung, die umfasst:
eine Eingabeeinheit (41) für persönliche physische Daten einer Person, deren Körpertyp bestimmt werden soll;
eine Impedanz-Messeinheit zum Messen einer bioelektrischen Impedanz der Person;
eine Berechnungseinheit (44) zum Berechnen eines Körpermasseindex (body mass index - BMI) und eines Körperzusammensetzungsindex (FMI, LMI) auf Basis der persönlichen physischen Daten und der bioelektrischen Impedanz der Person;
eine Speichereinheit (43) zum Vorspeichern einer Vielzahl von Regressionsformeln, wobei die Regressionsformeln Populationen als Objekten für Vergleichszwecke entsprechen und die Populationen nach physischen Daten klassifiziert werden;
eine Standard-Festlegeinheit zum Festlegen eines Normalwertes, der anhand einer Regressionsformel auf Basis gemessener Körpermasseindex-Daten und Körperzusammensetzungsindex-Daten ermittelt wird, die aus der Vielzahl von Regressionsformeln ausgewählt wird, als einen Standard zur Bestimmung des Körpertyps;
eine Körpertyp-Bestimmungseinheit zum Bestimmen des Körpertyps der Person auf Basis einer Differenz zwischen dem Normalwert, der aus der ausgewählten Regressionsformel ermittelt wird, und dem berechneten Körperzusammensetzungsindex (FMI, LMI) der Person; und
eine Bestimmungsstandard-Auswähltaste (41f), die eine manuelle Auswahl einer anderen Population als eines spezifischeren Objektes zu Vergleichszwecken ermöglicht, wenn sie betätigt wird, und wobei eine Regressionsformel, die einer Population als Objekt für Vergleichszwecke entsprechend Geschlecht und Alter der Person entspricht, deren Körpertyp bestimmt werden soll, automatisch ausgewählt wird, wenn die Bestimmungsstandard-Auswähltaste (41f) nicht betätigt wird, wobei die Vorrichtung so konfiguriert ist, dass der Körpertyp auf Basis der automatisch ausgewählten Regressionsformel bestimmt wird, danach eine Eingabe von der Person mittels der Bestimmungsstandard-Auswähltaste (41f) erwartet wird, und nach der Eingabe von der Person der Körpertyp auf Basis einer Regressionsformel bestimmt wird, die der manuell ausgewählten Population entspricht.

2. Körpertyp-Bestimmungsvorrichtung nach Anspruch 1, wobei die Berechnungseinheit (44) den Körpermasseindex (BMI) und wenigstens einen Fettmasseindex (fat mass index - FMI) oder einen Index der fettfreien Körpermasse (lean mass index - LMI) auf Basis der persönlichen physischen Daten und der bioelektrischen Impedanz der Person berechnet.

3. Körpertyp-Bestimmungsvorrichtung nach Anspruch 1 oder 2, wobei die Standard-Festlegeinheit eine Anzahl von Körpertyp-Bestimmungsstandards für verschiedene Objekte für Vergleichszwecke festlegt.

4. Körpertyp-Bestimmungsvorrichtung nach einem der Ansprüche 1 bis 3, wobei die Körpertyp-Bestimmungseinheit einen normalen Bereich oder einen abnormalen Bereich eines Körpertyps auf Basis wenigstens einer statistischen Methode aus einem Prozentwert oder einer Standardabweichung des Körperzusammensetzungsindex auf Basis des Normalwertes oder eines Z-Score auf Basis der Standardabweichung festlegt und den Körpertyp der Person nach einem Bereich bestimmt, zu dem Körperzusammensetzungsindex gehört.

5. Verfahren zum Bestimmen eines Körpertyps, das umfasst:
Eingeben persönlicher physischer Daten einer Person, deren Körpertyp zu bestimmen ist, mit einer Eingabeeinheit (41);
Messen einer bioelektrischen Impedanz der Person mit einer Impedanzmesseinheit;
Berechnen eines Körpermasseindex (body mass index - BMI) und eines Körperzusammensetzungsindex (body composition index - FMI, LMI) auf Basis der persönlichen physischen Daten und der bioelektrischen Impedanz der Person mit einer Berechnungseinheit (44);
Vorspeichern einer Vielzahl von Regressionsformeln mit einer Speichereinheit (43), wobei die Regressionsformeln Populationen als Objekte für Vergleichszwecke entsprechen und die Populationen nach physischen Daten klassifiziert werden;
Festlegen eines Normalwertes, der anhand einer Regressionsformel auf Basis gemessener Körpermasseindex-Daten und Körperzusammensetzungsindex-Daten ermittelt wird, die automatisch aus der Vielzahl von Regressionsformeln entsprechend Geschlecht und Alter der Person ausgewählt wird, wenn eine Bestimmungsstandard-Auswähltaste (41f) nicht eingesetzt wird, als einen Körpertyp-Bestimmungsstandard;
Bestimmen des Körpertyps der Person auf Basis einer Differenz zwischen dem anhand der ausgewählten Regressionsformel ermittelten Normalwert und dem berechneten Körperzusammensetzungsindex (FMI, LMI) der Person mit einer Körpertyp-Bestimmungseinheit; und
Auswählen einer anderen Population als ein spezifischeres Objekt für Vergleichszwecke, wenn die Bestimmungsstandard-Auswähltaste (41f) eingesetzt wird, wobei der Körpertyp auf Basis der automatisch ausgewählten Regressionsformel bestimmt wird, gefolgt von einem Schritt des Erwartens einer Eingabe von der Person mittels der Bestimmungsstandard-Auswähltaste (41f) und einem Schritt des Bestimmen des Körpertyps auf Basis einer Regressionsformel, die der manuell ausgewählten Population entspricht, nach der Eingabe von der Person.

## Revendications

1. Appareil de détermination de type de corps comprenant :
une unité d'entrée (41) pour les données physiques personnelles d'un sujet dont le type de corps doit être déterminé ;
une unité de mesure d'impédance pour mesurer l'impédance bioélectrique du sujet ;
une unité de calcul (44) pour calculer un indice de masse corporelle (BMI) et un indice de composition du corps (FMI, LMI) basés sur les données physiques personnelles et sur l'impédance bioélectrique du sujet ;
une unité de mémorisation (43) pour mémoriser à l'avance une pluralité de formules de régression, lesdites formules de régression correspondant à des populations en tant qu'objets de comparaison, lesdites populations étant classées par données physiques ;
une unité de réglage de standard pour régler une valeur normale obtenue à partir d'une formule de régression, basée sur les données d'indice de masse corporelle mesurée et sur les données d'indice de composition du corps, qui est choisie à partir de la pluralité de formules de régression, en tant que standard de détermination de type de corps ;
une unité de détermination de type de corps pour déterminer le type de corps du sujet en se basant sur la différence entre la valeur normale obtenue d'après la formule de régression sélectionnée et l'indice de composition du corps calculé (FMI, LMI) du sujet ; et
une clé de sélection de standard de détermination (41f), permettant, si elle est actionnée, une sélection manuelle d'une autre population en tant qu'objet de comparaison plus spécifique, et dans lequel une formule de régression correspondant à une population en tant qu'objet de comparaison fonction du sexe et de l'âge du sujet, dont le type de corps doit être déterminé, est sélectionnée automatiquement si la clé de sélection de standard de détermination (41f) n'est pas actionnée,
où l'appareil est configuré pour déterminer le type de corps en se basant sur la formule de régression sélectionnée automatiquement,
ce qui est suivi par l'attente d'une entrée de la part du sujet au moyen de la clé de sélection de standard de détermination (41f) et, lors de ladite entrée de la part du sujet, la détermination du type de corps en se basant sur une formule de régression correspondant à la population sélectionnée manuellement.

2. Appareil de détermination de type de corps selon la revendication 1, dans lequel l'unité de calcul (44) calcule l'indice de masse corporelle (BMI) et au moins un indice parmi un indice de masse grasse (FMI) et un indice de masse maigre (LMI) en se basant sur les données physiques personnelles et sur l'impédance bioélectrique du sujet.

3. Appareil de détermination de type de corps selon la revendication 1 ou 2, dans lequel l'unité de réglage de standard règle un certain nombre de standards de détermination de type de corps pour comparer différents objets.

4. Appareil de détermination de type de corps selon l'une des revendications 1 à 3, dans lequel l'unité de détermination de type de corps règle une plage convenable ou une plage anormale d'un type de corps en se basant sur au moins une technique statistique parmi une valeur de centile ou un écart type de l'indice de composition du corps, sur la base de la valeur normale ou d'une note Z basée sur l'écart type, et détermine le type de corps du sujet par une plage à laquelle appartient l'indice de composition du corps

5. Procédé de détermination de type de corps comprenant :
l'entrée, par une unité d'entrée (41), des données physiques personnelles d'un sujet dont le type de corps doit être déterminé ;
la mesure de l'impédance bioélectrique du sujet, par une unité de mesure d'impédance ;
le calcul d'un indice de masse corporelle (BMI) et d'un indice de composition du corps (FMI, LMI) basés sur les données physiques personnelles et sur l'impédance bioélectrique du sujet, par une unité de calcul (44) ;
la mémorisation à l'avance d'une pluralité de formules de régression, par une unité de mémorisation (43), lesdites formules de régression correspondant à des populations en tant qu'objets de comparaison, lesdites populations étant classées par données physiques ;
le réglage d'une valeur normale obtenue à partir d'une formule de régression basée sur les données d'indice de masse corporelle mesurée et sur les données d'indice de composition du corps, qui est choisie automatiquement à partir de la pluralité de formules de régression, en fonction du sexe et de l'âge du sujet si une clé de sélection de standard de détermination (41f) n'est pas actionnée, en tant que standard de détermination de type de corps ;
la détermination du type de corps du sujet basée sur la différence entre la valeur normale obtenue d'après la formule de régression sélectionnée et l'indice de composition du corps calculé (FMI, LMI) du sujet, par une unité de détermination de type de corps ; et
la sélection d'une autre population en tant qu'objet de comparaison plus spécifique, si la clé de sélection de standard de détermination (41f) est actionnée,
où le type de corps est déterminé en se basant sur la formule de régression sélectionnée automatiquement,
suivie par une étape d'attente d'une entrée de la part du sujet au moyen de la clé de sélection de standard de détermination (41f) et une étape de détermination du type de corps en se basant sur une formule de régression correspondant à la population sélectionnée manuellement lors de ladite entrée de la part du sujet.
